# EUROPÄISCHE PATENTANMELDUNG

(11) **EP 1 170 301 A1**
(43) Veröffentlichungstag der Anmeldung: **09.01.2002**
(21) Anmeldenummer: 01102668.9
(22) Anmeldetag: 26.06.1996
(51) Int. Cl.: C07K 1/06, C12P 21/02

(54) **Verfahren zur Herstellung von Peptiden und N-Carbamoyl-geschützten Peptiden**

(30) Priorität: 11.07.1995 DE 19524710; 05.02.1996 DE 19603844; 26.02.1996 DE 19607100
(62) Teilanmeldung aus: 96923933.4
(71) Anmelder: Degussa-Hüls Aktiengesellschaft, 60287 Frankfurt am Main (DE)
(72) Erfinder: Bommarius, Andreas, 30327 Atlanta, GA (US); Drauz, Karlheinz, 63579 Freigericht (DE); Eichhorn, Uwe, Dr., 01324 Dresden (DE); Jakubke, Hans-Dieter, Prof.Dr., 01465 Langebrück (DE); Kottenhahn, Matthias, 2920 Kalmthout (BE)

(57) **Zusammenfassung**

Es wird ein Verfahren zur enzymatischen Herstellung von geschützten Di- und Oligopeptiden beschrieben sowie die Abspaltung der verwendeten Schutzgruppen. Das erfindungsgemäße Verfahren erlaubt eine einfache und kostengünstige Synthese von Peptiden sowie eine schonende Abspaltung der Schutzgruppe. Das Verfahren beinhaltet drei Reaktionsschritte:
1. Darstellung der N-Carbamoyl-Aminosäure bzw. des N-Carbamoyl-Aminosäurederivates,
2. Knüpfung der Peptidbindung zwischen Carbamoylgeschütztem Elektrophil und Nukleophil sowie
3. Abspaltung der Carbamoyl-Schutzgruppe.

## Beschreibung

Die Erfindung betrifft ein Verfahren zur enzymatischen Herstellung von geschützten Di- oder Oligopeptiden und die Abspaltung der verwendeten Schutzgruppe.

Synthetische kurzkettige Peptide finden in der Pharmakologie und in der parenteralen Ernährung zunehmend Verwendung. Als Beispiel für ein pharmakologisch wirksames Dipeptid sei Kyotorphin (L-Tyr-L-Arg) genannt, das die Freisetzung von Enkephalinen fördert, endogenen Substanzen also, die im Gehirn eine analgetische und beruhigende Wirkung besitzen (Hughes, 1975).

Die enzymatische Herstellung von Di- oder Oligopeptiden ist bereits bekannt und bedient sich generell der Schutzgruppentechnologie. So wird in US-A-571,037 die Verwendung einer Formylschutzgruppe, in JP 62074296 einer Acetylschutzgruppe, in US-A-4,935,355 einer Benzylschutzgruppe oder in Tetrahedron 1992, 48, 1115 einer Phenacetylschutzgruppe beschrieben. Diese Varianten haben den Nachteil, daß die Schutzgruppen z. T. nicht kostengünstig sind (Benzyl, Phenacetyl), nur schwer wieder abspaltbar (Acetyl, Formyl) oder nur unter ganz bestimmten Bedingungen (Hydrogenolyse) entfernt werden können (Benzyl).

Aufgabe der Erfindung war daher, ein Verfahren zur Synthese von Peptiden zu entwickeln, das besonders einfach und kostengünstig ist, eine einfache und schonende Abspaltung der Schutzgruppe ermöglicht sowie eine einfache Aufarbeitung und Abtrennung des Enzyms ermöglicht.

Diese Aufgabe der Erfindung zur Herstellung eines geschützten Peptides der allgemeinen Formel III 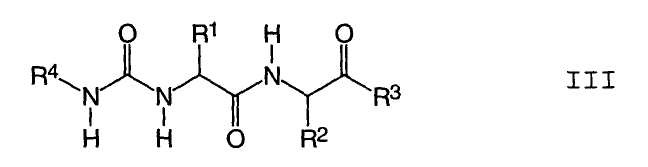 worin
- R¹ und R²: unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff, (C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
- R³: (C₁-C₄) Alkoxy, NH₂, Hydroxy, NR¹R², Benzyloxy, das ggf. ein- oder mehrfach durch Halogen, Nitro, NH₂, (C₁-C₄) Alkyl, (C₁-C₄) Alkoxy substituiert sein kann,
- R⁴: Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃, (C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeuten, wird gelöst, indem man eine Verbindung des Typs IV oder eine Salzform von IV, in der R¹ und R⁴ die oben angegebene Bedeutung besitzen, 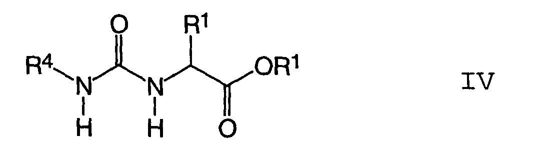 in Gegenwart von Hydrolasen,
wahlweise in Gegenwart eines Lösungsmittels und wahlweise in Gegenwart einer Base mit einer Verbindung des Typs V, oder eines Säureadditionssalzes hiervon, worin R² und R³ die oben angegebene Bedeutung besitzen, 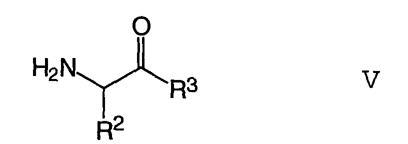 umsetzt.

Unter der Bezeichnung "Alkylgruppen" sind sowohl "geradkettige" als auch "verzweigte" Alkylgruppen zu verstehen.

Unter der Bezeichnung "geradkettige Alkylgruppe" sind beispielsweise Reste wie Methyl, Ethyl, n-Propyl, n-Butyl, n-Pentyl, n-Hexyl, unter "verzweigter Alkylgruppe" Reste wie beispielsweise Isopropyl oder tert.-Butyl zu verstehen.

Die Bezeichnung Halogen steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Bevorzugt können mit diesem Verfahren Dipeptide, wie beispielsweise Aspartam hergestellt werden.

Ein weiterer Vorteil des Verfahrens ist, daß man bei der Peptidkopplung in hochdichter Suspension arbeiten kann, wobei Substrat und Produkt in teilweiser fester Form vorliegen können.

Das neue Verfahren zur Synthese von Peptiden unter Verwendung der beschriebenen Carbamoyl-geschützten Peptide als Zwischenprodukt, beinhaltet drei Reaktionsschritte.
A) Darstellung der N-Carbamoyl-Aminosäure bzw. des N-Carbamoyl-Aminosäurederivates
B) Knüpfung der Peptidbindung zwischen Carbamoylgeschütztem Elektrophil und Nukleophil
C) Abspaltung der Carbamoyl-Schutzgruppe

Die Darstellung der N-Carbamoyl-Aminosäure bzw. des N-Carbamoyl-Aminosäurederivates kann in an sich literaturbekannter Weise durchgeführt werden. Vorzugsweise erfolgt die Umsetzung der Aminosäure bzw. des Aminosäurederivates mit einem Isocyanat der allgemeinen Struktur VI,

**R**^{**1**}**―NCO** VI

wobei R¹ die oben angegebene Bedeutung hat, in einem Zweiphasensystem aus H₂O/organischem Lösungsmittel, wobei das organische Lösungsmittel beispielsweise Toluol, Chlorbenzol oder tert.Butylmethylether sein kann. Die Umsetzung erfolgt bei Temperaturen zwischen 0 °C und 120 °C, vorzugsweise zwischen 20 °C und 70 °C. Gegebenenfalls kann dem Reaktionsgemisch eine anorganische Base, wie beispielsweise NaOH, KOH, K₂CO₃, Na₂CO₃, Kaliumhydrogencarbonat oder Natriumhydrogencarbonat oder eine organische Base, wie beispielsweise Triethylamin oder Tributylamin zugesetzt werden. Das Phenylisocyanat der Struktur III wird vorteilhafterweise im geringen Überschuß verwendet.

Besonders bevorzugt sind Umsetzungen zu den N-Carbamoylgeschützten Aminosäuren bzw. Aminosäurederivaten mit einem Cyanat der Struktur VII,

**M**^{**⊕ ⊖**} **OCN** VII

wobei M^{⊕} für Na^{⊕}, K^{⊕}, Ag^{⊕}, Pb^{⊕} (OCN) oder Ammoniumionen, wie beispielsweise Tetraethylammonium, Tetrabutylammonium oder Benzyltriethylammonium, steht.

Die Umsetzung erfolgt in an sich bekannter Weise in wäßriger Lösung bei Temperaturen zwischen 0 °C - 120 °C, vorzugsweise zwischen 60 °C - 100 °C und ggf. unter Zusatz einer anorganischen Base, wie beispielsweise NaOH, KOH, K₂CO₃ oder Na₂CO₃. Als günstig erweist sich, wenn die Reaktion in Gegenwart von leichten Überschüssen von 1.01 - 2 Äquivalenten, vorzugsweise 1.1 - 1.2 Äquivalenten an Cyanat durchgeführt wird.

Die Knüpfung der Peptidbindung wird in an sich bekannter Weise mit Hilfe einer Hydrolase durchgeführt (Jakubke, Kuhl und Könnecke; Angew. Chem. 1985, Vol. 97, S. 79 - 87). Dabei ist es für die Reaktionsbedingungen unerheblich, ob die Peptidknüpfung kinetisch oder thermodynamisch kontrolliert abläuft. Das erfindungsgemäße Verfahren ist auf beide Verfahrensvarianten anwendbar. Unter der Bezeichnung "Salzform" des Verbindungstyps IV sind allgemein ionische Strukturen zu verstehen. So kann ein mögliches Kation, wie beispielsweise Na^{⊕}, K^{⊕}, Ag^{⊕} , Pb^{2⊕} oder ein Ammoniumion, wie beispielsweise Tetraethylammonium, Tetrabutylammonium oder Benzyltriethylammonium, eine negative Ladung am Verbindungstyp IV neutralisieren. Die negative Ladung kann dabei über das Molekül (Verbindungstyp IV) verteilt sein oder an einem Stickstoffatom oder der Carboxylgruppe in Form einer Carboxylatgruppe lokalisiert sein.

Überraschenderweise verläuft diese Umsetzung mit der für diese Reaktionssequenz neuen Carbamoylgruppe als Schutzgruppe in guten Raum-/Zeitausbeuten und ermöglicht eine einfache Isolation der Produkte. Ebenso überraschend ist breite Anwendbarkeit der Reaktion.

Unter einem Säureadditionssalz von V ist beispielsweise ein HCl-Salz, HBr-Salz oder ein H₂SO₄-Salz zu verstehen.

Die Abspaltung der Carbamoyl-Schutzgruppe kann auch chemisch erfolgen. Diese Art der Abspaltung von Carbamoylgruppen von Peptiden ist ebenfalls neu. Die Abspaltung kann prinzipiell mit jedem NO^{⊕}-Donor erfolgen. Besonders geeignet sind NaNO₂ oder NO^{⊕}BF₄^{⊖} . Die Umsetzung erfolgt wahlweise in wäßrigem Milieu und/oder einem inerten organischen Lösungsmittel, wie einem aromatischen Kohlenwasserstoff, wie beispielsweise Toluol oder Chlorbenzol, in einem aliphatischen Kohlenwasserstoff, einem halogenierten Kohlenwasserstoff, wie beispielsweise Methylenchlorid oder einem Ether, wie beispielsweise Methyl-tert.butylether oder Tetrahydrofuran. Je nach NO^{⊕}-Donor wird die Reaktion in Gegenwart einer Säure, wie beispielsweise HCl oder H₂SO₄ durchgeführt. Als Reaktionstemperaturen eignen sich besonders Temperaturen zwischen +120 °C und -30 °C, besonders bevorzugt +60 °C und -20 °C, ganz besonders bevorzugt +25 °C und 0 °C. Bei Säurezugabe sollte der pH-Wert zwischen -0.5 und 5, bevorzugt 0 und 2, sein. Die Umsetzung ist deswegen vorteilhaft, da sie neben Stickstoff, CO₂ und ggf. einem Salz keinerlei andere Nebenprodukte liefert und der Umsatz mit hohen Ausbeuten und die gewünschte Produktbildung ohne Spaltprodukte verläuft. Ein weiterer Vorteil der Reaktion ist die einfache Aufarbeitung.

Aus der behandelten Peptidlösung kann das nun ungeschützte Peptid durch Kristallisation mittels Eindampfung, Kühlung oder ggf. Zusatz von organischen Lösungsmitteln ausgefällt und isoliert werden.

Das neue Verfahren wird anhand der folgenden Ausführungsbeispiele näher erläutert, ist aber nicht auf diese beschränkt.

### Beispiel 1

Exemplarische Darstellung von N-Carbamoyl-Aminosäuren und Derivaten am Beispiel der N-Carbamoyl-L-Asparaginsäure 12 g (0,3 mol) NaOH-Plätzchen werden in ca. 200 ml H₂O vorgelegt und gelöst. Die Asparaginsäure (33,3 g, 0,25 mol) wird in 50 ml Wasser gelöst zugegeben und das Reaktionsgemisch auf 80 °C erwärmt. Der pH-Wert liegt bei 8.7. Innerhalb von 5 min wird 16,6 g (0,255 mol) Natriumcyanat zugegeben (pH-Wert steigt auf 9.3, T auf 86 ° C). Nach 1 h Reaktionszeit ist keine Asparaginsäure mehr nachzuweisen.

### Beispiele zur Synthese der Peptide:

Die Aminosäuren und Peptide werden nach den international gültigen Regeln abgekürzt (IUPAC-JUB Joint Commission on Biochemical Nomenclature (JCBN); Nomenclature and Symbolism for Amino Acids and Peptides. Eur. J. Biochem. 158, 9 - 37 (1984)).
AC-: Aminocarbonyl- (Carbamoyl-, NH₂CO-)

### Beispiel 2: Synthese von AC-Asp-Phe-OMe

50 mg (0,2 mmol) AC-Asp(OK)-OK und 83 mg (0,4 mmol) H-Phe-OME•HCl werden in einem verschließbaren Polypropylengefäß mit 80 µl Puffer (0,5 M Hepes/Na⁺; pH 7) versetzt und sofort intensiv mit einem Spatel durchmischt. Der Reaktionsansatz wird in einem Wasserbad auf 40 °C thermostatiert und die Reaktion anschließend mit 20 µl Puffer, die 4 mg Thermolysinpräparation (Sigma P 1512) enthalten, gestartet. Nach 1 h, 2,5 h, 16 h, 22 h, 29 h und 39 h werden Proben entnommen und in 0,75 ml 4 %iger wäßriger Essigsäure abgestoppt. Die analytische Auswertung erfolgt hier wie in den folgenden Beispielen mittels HPLC und Vergleich mit einer authentischen Probe.

Ausbeute:

| Zeit (h) | Ausbeute (% d. Th.) | H-Phe-OH/H-Phe-OMe(t=0) (-/-) |
|---|---|---|
| 1 | 7 | 0,023 |
| 2,5 | 14 | 0,030 |
| 16 | 55 | 0,083 |
| 22 | 69 | 0,119 |
| 29 | 77 | 0,12 |
| 39 | 81 | 0,125 |

### Beispiel 3 a - e

Analog Beispiel 2 werden statt 80 µl Puffer 120 (a), 160 (b), 200 (c), 240 (d) und 280 µl Puffer zum Suspendieren der Reaktanden verwendet. Die Probennahme erfolgt nach 29 bzw. 39 h.

Ausbeute:

| Beispiel | Ausbeute (% d. Th.) | | H-Phe-OH/H-Phe-OMe(t=0) (-/-) | |
|---|---|---|---|---|
| | 29 h | 39 h | 29 h | 39 h |
| 3a | 79 | 82 | 0,139 | 0,136 |
| 3b | 79 | 76 | 0,144 | 0,165 |
| 3c | 73 | 75 | 0,169 | 0,169 |
| 3d | 64 | 64 | 0,216 | 0,22 |
| 3e | 59 | 61 | 0,215 | 0,224 |

### Beispiel 4

44 mg (0,175 mmol) AC-Asp(OK)-OK und 41,5 mg (0,2 mmol) H-Phe-OMe•HCl werden in einem verschließbaren Polypropylengefäß mit 80 µl Puffer (0,5 M Hepes/Na⁺; pH 7) versetzt und sofort intensiv mit einem Spatel suspendiert. Nach der Überführung des Reaktionsgefäßes in ein auf 40 °C thermostatiertes Wasserbad wird die Reaktion durch Zugabe von 15 µl Puffer, die 1,5 mg Thermolysinpräparation (Sigma P 1512) enthalten, gestartet. Nach 1 h, 2,5 h, 5 h, 6,5 h und 22 h werden Proben entnommen und in 0,75 ml 4 %iger wäßriger Essigsäure abgestoppt und mittels HPLC analysiert.

Ausbeute:

| Zeit (h) | Ausbeute (% d. Th.) | H-Phe-OH/H-Phe-OMe(t=0) (-/-) |
|---|---|---|
| 1 | 7 | 0,023 |
| 2,5 | 16 | 0,039 |
| 5 | 23 | 0,064 |
| 6,5 | 28 | 0,078 |
| 22 | 48 | 0,136 |

### Beispiel 5

Analog Beispiel 4 werden die Reaktanden statt mit 80 µl Puffer mit 10 (a), 30 (b), 50 (c), 130 (d) und 180 µl (e) Puffer versetzt. Die Probennahme erfolgt nach 22 h.

Ausbeute:

| Beispiel | Ausbeute (% d. Th.) | H-Phe-OH/H-Phe-OMe(t=0) (-/-) |
|---|---|---|
| 5a | 7 | 0,034 |
| 5b | 31 | 0,072 |
| 5c | 29 | 0,105 |
| 5d | 39 | 0,319 |
| 5e | 41 | 0,249 |

### Beispiel 6: Synthese von AC-Asp-Phe-NH₂

250 mg (1.0 mmol) AC-Asp(OK)-OK und 400 mg (2.0 mmol) H-Phe-NH₂•HCl werden in einem verschließbaren Polypropylengefäß mit 400 µl Puffer (0,5 M Hepes/Na⁺; ph 7) versetzt und sofort intensiv mit einem Spatel suspendiert. Nach der Überführung des Reaktionsgefäßes in ein auf 40 °C thermostatiertes Wasserbad wird die Reaktion durch Zugabe von 50 µl Puffer, die 10 mg Thermolysinpräparation (Sigma P 1512) enthalten, gestartet. Nach 44 h werden Proben entnommen und in 0,75 ml 4 %iger wäßriger Essigsäure abgestoppt und mittels HPLC analysiert. Die Reaktion wird durch Zugabe von 1 ml 1 N wäßrige HCl beendet und über Nacht bei 4 °C belassen. Anschließend wird der Niederschlag abgesaugt und mit eiskaltem Wasser gewaschen.
Ausbeute: 62 % d. Th.
Schmelzbereich: 221 - 223 °C
¹H-NMR (D₆-DMSO, 300 MHz): δ=2,38-2,6 (m, 2 H, -^{β}CH₂-), δ=2,8-2,88 (m, 1 H, -^{β}CH₂-), δ=5,72 (s, 2 H, -NH₂), δ=6,30 (d, 1 H, -^{α}NH-, J = 7,69), δ=7,13 (s, 1 H, Phe-NH₂), δ=7,26-7,28 (m, 5 H, Phe-C₆H₅), δ=7,33 (s, 1 H, Phe-NH₂), δ=7,83 (d, 1 H, -^{α} NH-, J = 8,4)

### Beispiel 7: Synthese von AC-Asp-Phe-Leu-NH₂

67,4 mg (0,2 mmol) AC-Asp-Phe-OMe und 26 mg (0,2 mmol) H-Leu-NH₂ (Bachem Biochemica GmbH) werden in einem Polypropylenreaktionsgefäß in 180 µl Puffer (0,5 M Hepes/Na⁺, pH 7,9) gelöst. Nach Zugabe von 10 µl 10 M wäßriger NaOH wird die Reaktion mit 10 µl α-Chymotrypsinlösung (10 mg/ml Puffer; Serva, 3 x krist.) gestartet. Nach 2, 5, 10, 20, 30 und 60 min werden Proben entnommen, in 1 ml 4 %iger wäßriger Essigsäure gelöst und mittels HPLC analysiert. Der verbliebene Reaktionsansatz wird in 1 ml Methanol aufgenommen , filtriert und das Filtrat mit 1 ml 1 N wäßriger HCl und 4 ml Wasser versetzt. AC-Asp-Phe-Leu-NH₂ kristallisierte innerhalb von 48 h aus. Die Kristalle wurden abgetrennt und 2 mal mit eiskaltem Wasser (je ca. 1,5 ml) gewaschen und anschließend über P₄O₁₀ im Vakuum getrocknet.

Ausbeute:

| Zeit (min) | AC-Asp-Phe-OH (% d. Th) | AC-Asp-Phe-Leu-NH₂ (% d. Th.) |
|---|---|---|
| 2 | 18,3 | 11,6 |
| 5 | 19 | 23,9 |
| 10 | 19,8 | 41,5 |
| 20 | 21,2 | 60,5 |
| 30 | 21,6 | 70,2 |
| 60 | 20,8 | 79,1 |

¹H-NMR;COSY(DMSO, 300 MHz): δ=0,82 (d, 3 H, Leu-^{δ}CH₃), δ=0,87 (d, 3 H, Leu-^{δ} CH₃), δ=1,41-1,6 (m, 3 H, Leu-^{γ}CH-, Leu-^{β}CH₂-), δ=2,35-2,58 (m, 2 H, Asp-^{β}CH₂-), δ=2,81-3,06 (m, 2 H, Phe-^{β}CH₂-), δ=4,18 (dt, 1 H, Leu-^{α}CH-), δ=4,33 (dt, 1 H, Asp-^{α}CH-), δ=4,44 (dt, 1 H, Phe-^{α}CH-), δ=5,70 (s, 2 H, AC-NH₂), δ=6,30 (d, 1 H, Asp-^{α}NH-), δ=6,96 (s, 1 H, Leu-NH₂), δ=7,05 (s, 1 H, Leu-NH₂), δ=7,14-7,27 (m, 5 H, Phe-C₆H₅), δ=7,85-7,96 (m, 2 H, Leu-^{α}NH, Phe-^{α}NH)

### Beispiel 8: Synthese von AC-Asp-Phe-Ile-Gly-OMe

Analog Beispiel 7 werden statt H-Leu-NH₂ 0,2 mmol (47,8 mg) H-Ile-Gly-OMe**•**HCl eingesetzt. Zur pH-Wert-Einstellung werden 35 µl des Puffers durch 10 M wäßrige NaOH ersetzt.

Ausbeute nach 70 min:

| AC-Asp-Phe-OH (% d. Th.) | AC-Asp-Phe-Ile-Gly-OH (% d. Th.) | AC-Asp-Phe-Ile-Gly-OMe (% d. Th.) |
|---|---|---|
| 28,4 | 3,9 | 67,7 |

¹H-NMR;COSY(DMSO, 300 MHz): δ=0,77-0,88 (m, 6 H, Ile-^{δ}CH₃), δ=1,00-1,15 (m, 1 H, Ile-^{γ}CH₂-), δ=1,38-1,52 (m, 1 H, Ile-^{γ}CH₂-) , δ=1,65-1,78 (m, 1 H, Ile-^{β}CH-), δ=2,34-2,58 (m, 2 H, Asp-^{β}CH₂-), δ=2,78-2,88 (m, 1 H, Phe-^{β}CH₂-), δ=2,94-3,2 (m, 1 H, Phe-^{β}CH₂-), δ=3,62 (s, 3 H, OCH₃), δ=3,75-3,95 (m, 2 H, Gly-^{α}CH₂-), δ=4,17 (t, 1 H, Ile-^{α}CH-), δ=4,30-4,40 (m, 1 H, Asp-^{α}CH-), δ=4,48-4,58 (m, 1 H, Phe-^{α}CH-), δ=5,7 (s-breit, AC-NH₂), δ=6,30 (d, 1 H, Asp-^{α}NH-), δ=7,12-7,26 (m, 5 H, Phe-C₆H₅-), δ=7,80 (d, 1 H, Phe-^{α}NH-), δ=7,92 (d, 1 H, Leu-^{α}NH-), δ=8,32 (t, 1 H, Gly-^{α}NH-).

### Beispiel 9: Synthese von AC-Tyr-Ile-Ala-NH₂

a) 44,8 mg (0,2 mmol) AC-Tyr-OH und 47,4 mg (0,2 mmol) H-Ile-Ala-NH₂•HCl werden in einem verschließbaren Polypropylengefäß mit 45 µl Puffer (0,5 M Hepes/Na⁺; pH 7) versetzt und sofort intensiv mit einem Spatel suspendiert. Die pH-Wert-Einstellung erfolgt durch Zugabe von 25 µl 10 M wäßriger NaOH. Nach der Überführung des Reaktionsgefäßes in ein auf 40 °C thermostatiertes Wasserbad wird die Reaktion durch Zugabe von 10 µl Puffer, die 1 mg Thermolysinpräparation (Sigma P 1512) enthalten, gestartet. Nach 3 h wird eine Probe entnommen und in 1 ml 4 %iger wäßriger Essigsäure und 200 µl Acetonitril abgestoppt und mittels HPLC analysiert. Die Aufarbeitung des verbliebenen Reaktionsansatzes erfolgt wie in Beispiel 7 mit dem Unterschied, daß statt 1 N HCl 4 %ige wäßrige Essigsäure verwendet wird.
   Ausbeute an AC-Tyr-Ile-Ala-NH₂ nach 3 h: 76 % d. Th.
   ¹H-NMR;COSY(DMSO, 300 MHz): δ=0,76-0,84 (m, 6 H, Ile-^{δ} CH₃, Ile-^{γ}CH₃), δ=1,00-1,12 (m, 1 H, Ile-^{γ}CH₂-), δ=1,22 (d, 3 H, Ala-^{β}CH₃), δ=1,36-1,42 (m, 1 H, Ile-^{γ}CH₂-), δ=1,68-1,78 (m, 1 H, Ile-^{β}CH-), δ=2,56-2,68 (m, 1 H, Tyr-^{β}CH₂-), δ=2,80-2,90 (m, 1 H, Tyr-^{β}CH₂-), δ=4,12-4,24 (m, 2 H, Ala-^{α}CH-, Ile-^{α} CH-), δ=4,28-4,38 (m, 1 H, Tyr-^{α}CH-), δ=5,58 (s, 2 H, AC-NH₂), δ=6,03 (d, 1 H, Tyr-^{α}NH-), δ=6,61 (d, 2 H, Tyr-ArH), δ=6,92-6,98 (m, 3 H, 2 Tyr-ArH, 1 Ala-NH₂), δ=7,20 (s, 1 H, Ala-NH₂), δ=7,84 (d, 1 H, Ala-^{α}NH-), δ=7,92 (d, 1 H, Ile-^{α}NH-), δ=9,12 (s, 1 H, Tyr-OH)
b) Analog Beispiel 9a werden 224 mg (1.0 mmol) AC-Tyr-OH, 237,7 mg (1,0 mmol) H-Ile-Ala-NH₂•HCl , 225 µl Puffer, 125 µl 10 M NaOH und 50 µl Thermolysinsuspension, die 5 mg Thermolysin (Sigma P1512) enthalten, eingesetzt.
   Ausbeute an AC-Tyr-Ile-Ala-NH₂ nach 16 h:
   228 mg (= 56 % d. Th.)

### Beispiel 10: Synthese von AC-Tyr-Val-NH₂

Analog Beispiel 9a werden statt H-Ile-Ala-NH₂•HCl 31,2 mg (0,2 mmol) H-Val-NH₂•HCl eingesetzt.
Ausbeute an AC-Tyr-Val-NH₂ nach 18 h: 65 % d. Th.
¹H-NMR (D₆-DMSO, 300 MHz): δ=0,78-0,88 (m, 6 H, Val-^{γ} CH₃), δ=1,92-2,00 (m, 1 H, Val-^{β}CH-), δ=2,58-2,68 (m, 1 H, Tyr-^{β}CH₂-), δ=2,80-2,90 (m, 1 H, Tyr-^{β}CH₂-), δ=4,11 (dd, 1 H, Val-^{α}CH-), δ=4,28-4,38 (m, 1 H, Tyr-^{α}CH-), δ=5,57 (s, 2 H, AC-NH₂), δ=6,08 (d, 1 H, Tyr-^{α}NH-), δ=6,62 (d, 2 H, Tyr-ArH), δ=6,97 (d, 2 H, Tyr-ArH), δ=7,03 (s, 1 H, Val-NH₂), δ=7,31 (s, 1 H, Val-NH₂), δ=7,67 (d, 1 H, Val-^{α}NH-), δ=9,12 (s, 1 H, Tyr-OH)

### Beispiel 11: Synthese von AC-Tyr-Met-NH₂

Analog Beispiel 9a werden statt H-Ile-Ala-NH₂•HCl 37 mg (0,2 mmol) H-Met-NH₂•HCl eingesetzt.
Ausbeute an AC-Tyr-Met-NH₂ nach 14 h: 75 % d. Th.
¹H-NMR (D₆-DMSO, 300 MHz): δ=1,75-1,85 (m, 1 H, Met-^{β}CH₂-), δ=1,88-2,00 (m, 1 H, Met-^{β}CH₂-), δ=2,02 (s, 3 H, Met-S-CH₃), δ=2,30-2,45 (m, 2 H, Met-^{β}CH₂-), δ=2,60-2,85 (m, 2 H, Tyr-^{β}CH₂-), δ=4,15-4,28 (m, 2 H, Tyr-^{α}CH-, Met-^{α}CH-), δ=5,65 (s, 2 H, AC-NH₂), δ=6,28 (d, 1 H, Tyr-^{α}NH-), δ=6,63 (d, 2 H, Tyr-ArH), δ=6,97 (d, 2 H, Tyr-ArH), δ=7,04 (s, 1 H, Met-NH₂), δ=7,25 (s, 1 H, Met-NH2), δ=8,08 (d, 1 H, Met-^{α}NH-), δ=8,54 (s, 1 H, Tyr-OH)

### Beispiel 12: Synthese von AC-Tyr-Nvl-NH₂

Analog Beispiel 9a werden statt H-Ile-Ala-NH₂•HCl 31,2 mg (0.2 mmol) H-Hvl-NH₂•HCl eingesetzt.
Ausbeute an AC-Tyr-Nvl-NH₂ nach 14 h: 88 % d. Th.
¹H-NMR (D₆-DMSO, 300 MHz): δ=0,85 (t, 3 H, Nvl-^{δ} CH₃), δ=1,18-1,32 (m, 2 H, Nvl-^{γ}CH₂-), δ=1,45-1,68 (m, 2 H, Nvl-^{β}CH₂-), δ=2,55-2,68 (m, 1 H, Tyr-^{β}CH₂-), δ=2,78-2,88 (m, 1 H, Tyr-^{β} CH₂-), δ=4,12-4,30 (m, 2 H, Tyr-^{α}CH-, Nvl-^{α}CH-), δ=5,59 (s, 2 H, AC-NH₂), δ=6,02 (d, 1 H, Tyr-^{α}NH-), δ=6,62 (d, 2 H, Tyr-ArH), δ=6,94-6,99 (m, 3 H, 2 Tyr-ArH, 1 Nvl-NH₂), δ=7,21 (s, 1 H, Nvl-NH₂), δ=7,79 (d, 1 H, Met-^{α}NH-), δ=9,12 (s, 1 H, Tyr-OH)

### Beispiel 13: Synthese von AC-Tyr-Phe-NH₂

Analog Beispiel 9a werden statt H-Ile-Ala-NH₂•HCl 40 mg (0,2 mmol) H-Phe-NH₂•HCl eingesetzt.
Ausbeute an AC-Tyr-Phe-NH₂ nach 1 h: 86 % d. Th.
¹H-NMR (D6-DMSO, 300 MHz): δ=2,72-2,88 (m, 3 H, -^{β}CH₂-), δ=1,98-3,06 (m, 1 H, -^{β}CH₂-), δ=4,12-4,20 (m, 1 H, -^{α}CH-), δ=4,38-4,46 (m, 1 H, -^{α}CH-), δ=5,59 (s, 2 H, AC-NH₂), δ=5,97 (d, 1 H, Tyr-^{α}NH-), δ=6,61 (d, 2 H, Tyr-ArH), δ=6,92 (d, 2 H, Tyr-ArH), δ=7,08 (s, 1 H, Phe-NH₂), δ=7,15-7,28 (m, 5 H, Phe-C₆H₅), δ=7,32 (s, 1 H, Phe-NH₂), δ=7,92 (d, 1 H, Phe-^{α}NH-), δ=9,12 (s, 1 H, Tyr-OH)

### Beispiel 14: Synthese von AC-Tyr-Phe-OMe

Analog Beispiel 9a werden statt H-Ile-Ala-NH₂•HCl 43 mg (0,2 mmol) H-Phe-OMe•HCl eingesetzt. Die verwendete Thermolysinmenge beträgt 2 mg.
Ausbeute an AC-Tyr-Phe-OMe nach 14 h: 81 % d. Th.
¹H-NMR (D₆-DMSO, 300 MHz): δ=2,50-2,62 (m, 1 H, -^{β}CH₂-), δ=2,72-2,85 (m, 1 H, -^{β}CH₂-), δ=2,90-3,08 (m, 2 H, -^{β}CH₂-), δ=3,58 (s, 3 H, -O-CH₃), δ=4,25-4,35 (m, 1 H, -^{α}CH-), δ=4,44-4,52 (m, 1 H, -^{α}CH-), δ=5,52 (s, 2 H, AC-NH₂), δ=5,99 (d, 1 H, Tyr-^{α}NH-), δ=6,62 (d, 2 H, Tyr-ArH), δ=6,94 (d, 2 H, Tyr-ArH), δ=7,15-7,30 (m, 5 H, Phe-C₆H₅), δ=8,36 (d, 1 H, Phe-^{α} NH-), δ=9,14 (s, 1 H, Tyr-OH)

### Beispiel 15: Synthese von AC-Tyr-Ile-NH₂

Analog Beispiel 9a werden statt H-Ile-Ala-NH₂•HCl 33,2 mg (0,2 mmol) H-Ile-NH₂•HCl eingesetzt.
Ausbeute an AC-Tyr-Ile-NH₂ nach 18 h: 68,9 % d. Th.
¹H-NMR (D₆-DMSO, 300 MHz): δ=0,76-0,86 (m, 6 H, Ile-^{β}CH₃, Ile-^{γ}CH₃), δ=0,99-1,12 (m, 1 H, Ile-^{γ}CH₂-), δ=1,35-1,46 (m, 1 H, Ile-^{γ} CH₂-), δ=1,62-1,76 (m, 1 H, Ile-^{β}CH-), δ=2,58-2,68 (m, 1 H, Tyr-^{β}CH₂-), δ=2,79-2,88 (m, 1 H, Tyr-^{β} CH₂-), δ=4,12 (dd, 1 H, Ile-^{α}CH-) , δ=4,26-4,37 (m, 1 H, Tyr-^{α} CH-), δ=5,58 (s, 2 H, AC-NH₂), δ=6,06 (d, 1 H, Tyr-^{α}NH-), δ=6,62 (d, 2 H, Tyr-ArH), δ=6,96 (d, 2 H, Tyr-ArH), δ=7,02 (s, 1 H, Ile-NH₂), δ=7,31 (s, 1 H, Ile-NH₂), δ=7,68 (d, 1 H, Ile-^{α}NH-), δ=9,12 (s, 1 H, Tyr-OH)

### Beispiel 16: Synthese von AC-Tyr-Leu-NH₂

Analog Beispiel 9a werden 224 mg (1,0 mmol) AC-Tyr-OH, 130 mg (1.0 mmol) H-Leu-NH₂ (Bachem Feinchemikalien AG), 225 µl Puffer, keine NaOH und 50 µl Thermolysinsuspension, die 5 mg Thermolysin (Sigma P1512) enthalten, eingesetzt.
Ausbeute an AC-Tyr-Leu-NH₂ nach 16 h:
127,8 mg (= 38 % d. Th.)
¹H-NMR (D₆-DMSO, 300 MHz): δ=0,80-0,92 (m, 6 H, Leu-^{δ} CH₃), δ=1,42-1,62 (m, 3 H, Leu-^{γ}CH-, Leu-^{β}CH₂-), δ=2,58-2,70 (m, 1 H, Tyr-^{β}CH₂-), δ=2,79-2,88 (m, 1 H, Tyr-^{β}CH₂-), δ=4,16-4,30 (m, 2 H, Leu-^{α} CH-, Tyr-^{α}CH-), δ=5,60 (s, 2 H, AC-NH₂), δ=6,02 (d, 1 H, Tyr-^{α}NH-), δ=6,63 (d, 2 H, Tyr-ArH), δ=6,93-7,00 (m, 2 H, Tyr-ArH, 1 H, Leu-NH₂), δ=7,19 (s, 1 H, Leu-NH₂), δ=7,83 (d, 1 H, Leu-^{α}NH-), δ=9,15 (s, 1 H, Tyr-OH)

### Beispiel 17: Synthese von AC-Tyr-Ile-Gly-OMe

Analog Beispiel 9a werden statt H-Ile-Ala-NH₂•HCl 47,8 mg (0,2 mmol) H-Ile-Gly-OMe•HCl eingesetzt.
Ausbeute an AC-Tyr-Ile-Gly-OMe nach 3 h: 89 % d. Th.
¹H-NMR;COSY(D₆-DMSO, 300 MHz): δ=0,76-0,88 (m, 6 H, Ile-^{δ}CH₃, Ile-^{γ}CH₃), δ=1,00-1,14 (m, 1 H, Ile-^{γ}CH₂-), δ=1,36-1,52 (m, 1 H, Ile-^{γ}CH₂-), δ=1,65-1,78 (m, 1 H, Ile-^{β}CH-), δ=2,55-2,68 (m, 1 H, Tyr-^{β}CH₂-), δ=2,75-2,86 (m, 1 H, Tyr-^{β}CH₂-), δ=3,62 (s, 3 H, -O-CH₃), δ=3,84 (t, 2 H, Gly-^{α}CH₂-), δ=4,27-4,35 (m, 1 H, Ile-^{α}CH-), δ=4,38-4,48 (m, 1 H, Tyr-^{α} CH-), δ=5,57 (s, 2 H, AC-NH₂), δ=6,05 (d, 1 H, Tyr-^{α}NH-), δ=6,61 (d, 2 H, Tyr-ArH), δ=6,95 (d, 2 H, Tyr-ArH), δ=7,79 (d, 1 H, Ile-^{α}NH), δ=8,32 (t, 1 H, Gly-^{α}NH-), δ=9,11 (s, 1 H, Tyr-OH)

### Beispiel 18: Synthese von AC-Tyr-Val-OBzl

Analog Beispiel 16 werden statt H-Leu-NH₂ 379,4 mg (1.0 mmol) H-Val-OBzl•pTos (Bachem Feinchemikalien AG), 175 µl Puffer, 125 µl 10 M wäßrige NaOH und 100 µl Thermolysinsuspension, die 10 mg Thermolysin (Sigma P1512) enthalten, eingesetzt.
Ausbeute an AC-Tyr-Val-OBzl nach 88 h:
230 mg (= 55 % d. Th.)
¹H-NMR (D₆-DMSO, 300 MHz): δ=0,81-0,92 (m, 6 H, Val-^{γ}CH₃), δ=2,01-2,14 (m, 1 H, Val-^{β}CH-), δ=2,50-2,64 (m, 1 H, Tyr-^{β}CH₂-), δ=2,75-2,85 (m, 1 H, Tyr-^{β}CH₂-), δ=4,23 (dd, 1 H, Val-^{α}CH-), δ=4,36-4,45 (m, 1 H, Tyr-^{α}CH-), δ=5,13 (s, 2 H, -O-CH₂-), δ=5,52 (s, 2 H, AC-NH₂), δ=6,04 (d, 1 H, Tyr-^{α}NH-), δ=6,62 (d, 2 H, Tyr-ArH), δ=6,96 (d, 2 H, Tyr-ArH), δ=7,30-7,40 (m, 5 H, -C₆H₅), δ=8,20 (d, 1 H, Val-^{α}NH-), δ=9,13 (s, 1 H, Tyr-OH)

### Beispiel 19: Synthese von AC-Tyr-Phe-Ala-OBzl

Analog Beispiel 9a werden statt H-Ile-Ala-NH₂•HCl 88 mg (0,2 mmol) H-Phe-Ala-OBzl•TFA eingesetzt.
Ausbeute an AC-Tyr-Phe-Ala-OBzl nach 3 h: 72 % d. Th.
¹H-NMR ;COSY(D₆-DMSO, 300 MHz): δ=1,33 (d, 3 H, Ala-^{β}CH₃), δ=2,48-2,58 (m, 1 H, Tyr-^{β}CH₂-), δ=2,70-2,85 (m, 2 H, Tyr-^{β}CH₂-), δ=2,95-3,05 (m, 1 H, Phe-^{β}CH₂-), δ=4,14-4,24 (m, 1 H, Tyr-^{α}CH-), δ=4,32-4,41 (m, 1 H, Ala-^{α}CH-), δ=4,51-4,62 (m, 1 H, Phe-^{α} CH-), δ=5,13 (s, 2 H, O-CH₂-), δ=5,56 (s, 2 H, AC-NH₂), δ=5,94 (d, 1 H, Tyr-^{α}NH-), δ=6,60 (d, 2 H, Tyr-ArH), δ=6,90 (d, 2 H, Tyr-ArH), δ=7,14-7,28 (m, 5 H, OBzl-C₆H₅), δ=7,30-7,40 (m, 5 H, Phe-C₆H₅), δ=8,01 (d, 1 H, Phe-^{α}NH), δ=8,46 (d, 1 H, Ala-^{α}NH-), δ=9,12 (s, 1 H, Tyr-OH)

### Beispiel 20: Synthese von AC-Met-Phe-NH₂

Analog Beispiel 9a werden statt AC-Tyr-OH 76,8 mg (0,2 mmol) AC-Met-OH 80 mg (0,4 mmol) H-Phe-NH₂•HCl (Degussa AG), 90 µl Puffer, 50 µl 10 M wäßrige NaOH und 20 µl Thermolysinsuspension, die 2 mg Thermolysin (Sigma P1512) enthalten, eingesetzt.
Ausbeute an AC-Met-Phe-NH₂ nach 3 h: 96 % d. Th.
¹H-NMR (D₆-DMSO, 300 MHz): δ=1,58-1,80 (m, 2 H, Met-^{β}CH₂-), δ=1,99 (s, 3 H, Met-S-CH₃), δ=2,25-2,36 (m, 2 H, Met-^{γ}CH₂-), δ=2,76-2,88 (m, 1 H, Phe-^{β}CH₂-), δ=2,98-2,08 (m, 1 H, Phe-^{β}CH₂-), δ=3,98-4,08 (m, 1 H, -^{α} CH-), δ=4,36-4,47 (m, 1 H, -^{α}CH-) , δ=5,63 (s, 2 H, AC-NH₂), δ=6,23 (d, 1 H, Met-^{α}NH-), δ=7,09 (s, 1 H, Phe-NH₂), δ=7,12-7,28 (m, 5 H, Phe-C₆H₅), δ=7,42 (s, 1 H, Phe-NH₂), δ=7,96 (d, 1 H, Phe-^{α}NH-)

### Beispiel 21: Synthese von AC-Leu-Phe-NH₂

Analog Beispiel 20 werden statt AC-Met-OH 69,6 mg (0,4 mmol) AC-Leu-OH eingesetzt.
Ausbeute an AC-Leu-Phe-NH₂ nach 70 h: 97 % d. Th.
¹H-NMR (D₆-DMSO, 300 MHz): δ=0,78-0,88 (m, 6 H, Leu-^{δ}CH₂-), δ=1,16-1,35 (m, 2 H, Leu-^{β}CH₂-, Leu-^{γ}CH-), δ=1,45-1,56 (m, 1 H, Leu-^{β}CH₂-), δ=2,78-2,90 (m, 1 H, Phe-^{β}CH₂-), δ=2,99-3,08 (m, 1 H, Phe-^{β}CH₂-), δ=3,90-4,00 (m, 1 H, -^{α}CH-), δ=4,36-4,45 (m, 1 H, -^{α}CH-), δ=5,58 (s, 2 H, AC-NH2), δ=6,08 (d, 1 H, Leu-^{α}NH-), δ=7,06 (s, 1 H, Phe-NH₂), δ=7,12-7,28 (m, 5 H, Phe-C₆H₅), δ=7,34 (s, 1 H, Phe-NH₂), δ=7,82 (d, 1 H, Phe-^{α}NH-)

### Beispiel 23

Abspaltung der Carbamoyl-Schutzgruppe (exemplarisch am Beispiel von Carbamoyl-L-Aspartam) durch Zusatz von Natriumnitrit

4.72 g (12.6 mmol) Carbamoyl-L-Aspartam-Kaliumsalz werden in 60 ml H₂O / 40 ml HCl (konz., technisch) vorgelegt, auf 5 °C abgekühlt (pH = 0.80) und 15.1 ml (15.1 mmol) NaNO₂-Lösung langsam (1 ml/10 min) zudosiert. Man läßt ca. 1 h nachreagieren, bevor das Reaktionsgemisch mit 50 Gew.-% Natronlauge neutralisiert wird. Das Reaktionsgemisch wird über Nacht bei RT belassen, bevor der ausgefallene Niederschlag abgetrennt und verworfen wird. Die Mutterlauge wird anschließend eingeengt bis Aspartam ausfällt. Das so gewonnene Aspartam wird im Vakuum bei 60 °C bis zur Gewichtskonstanz getrocknet. Die Umsetzung verläuft quantitativ.

## Patentansprüche

1. Verfahren zur Herstellung von Peptiden der allgemeinen Struktur III, 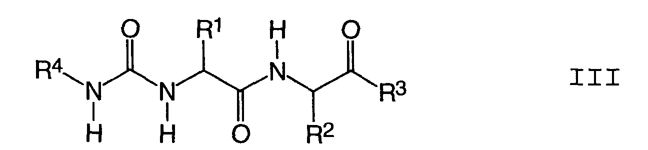 worin
R¹ und R² unabhängig voneinander Wasserstoff, (C₁-C₆) Alkyl, das ggf. ein oder mehrfach mit Heteroatome, wie N, O oder S, unterbrochen oder substituiert sein kann, wobei die Heteroatome ihrerseits mit Wasserstoff, (C₁-C₄) Alkyl oder Benzyl substituiert sein können oder über eine Doppelbindung mit der Alkylgruppe verbunden sein können, Phenyl oder Benzyl, die beide ggf. ein- oder mehrfach mit Halogen oder Hydroxy substituiert sein können, Heteroaralkyl, wie 3-Indolylmethyl, 2-, 3- oder 4-Pyridylmethyl,
R³ (C₁-C₄) Alkoxy, NH₂, Hydroxy, NR¹R², Benzyloxy, das ggf. ein- oder mehrfach durch Halogen, Nitro, NH₂, (C₁-C₄) Alkyl, (C₁-C₄) Alkoxy substituiert sein kann,
R⁴ Wasserstoff, (C₁-C₄) Alkyl, Phenyl, das ggf. ein- oder mehrfach mit Halogen, (C₁-C₄) Alkyl, (C₁-C₄) Alkoxy, Nitro, CN, CF₃, (C₁-C₆) Alkoxycarbonyl, COOH oder -NR¹R² substituiert sein kann, Aralkyl, wie Benzyl, das seinerseits mit Halogen, (C₁-C₄) Alkyl oder (C₁-C₄) Alkoxy substituiert sein kann, Naphthyl, Heteroaralkyl, wie 2-, 3- oder 4-Thienyl, 2-, 3- oder 4-Pyridyl oder 2-Chinolyl
bedeuten,
**dadurch gekennzeichnet**,
daß eine Verbindung des Typs IV oder eine Salzform von IV, in der R¹ und R⁴ die oben angegebene Bedeutung besitzen, 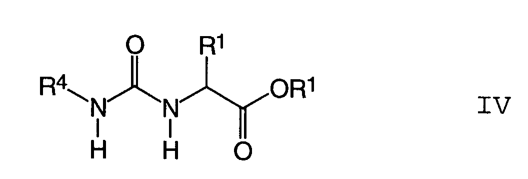 in Gegenwart von Hydrolasen, wahlweise in Gegenwart eines Lösungsmittels und wahlweise in Gegenwart einer Base mit einer Verbindung des Typs V oder eines Säureadditionssalzes hiervon, worin R² und R³ die oben angegebene Bedeutung besitzen, 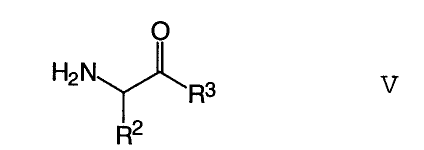 umgesetzt wird.

2. Verfahren nach Anspruch 1,
**dadurch gekennzeichnet**,
daß die Abspaltung der Carbamoyl-Schutzgruppe mit einem NO^{⊕}-Donor, bevorzugt mit NaNO₂ oder NO^{⊕}BF₄^{⊖} erfolgt.

3. Verfahren nach Anspruch 2,
**dadurch gekennzeichnet**,
daß die Abspaltung in einem Temperaturbereich zwischen +120 °C und -30 °C, vorzugsweise zwischen +60 °C und -20 °C, ganz besonders bevorzugt zwischen 25 °C und 0 °C, erfolgt.

4. Verfahren nach Anspruch 2,
**dadurch gekennzeichnet**,
daß die Umsetzung unter Säurezusatz in einem pH-Bereich zwischen -0.5 und 5, bevorzugt zwischen 0 und 2, erfolgt.

5. Verfahren nach Anspruch 2,
**dadurch gekennzeichnet**,
daß die Umsetzungen im wäßrigen Medium stattfinden.

6. Verfahren nach einem der vorangegangenen Ansprüche,
**dadurch gekennzeichnet**,
daß das gespaltene Peptidderivat Aspartam ist.

7. Verfahren nach Anspruch 1,
**dadurch gekennzeichnet**,
daß die Peptidkopplung in hochdichter Suspension erfolgt.

8. Verfahren nach Anspruch 7,
**dadurch gekennzeichnet**,
daß Substrat und Produkt in teilweise fester Form vorliegen.
